# EUROPEAN PATENT APPLICATION

(11) **EP 2 248 542 A2**
(43) Date of publication of application: **10.11.2010**
(21) Application number: 10158711.1
(22) Date of filing: 31.03.2010
(51) Int. Cl.: A61L 29/12, A61L 29/14

(54) **Polycarbonate polyurethane venous access devices having enhanced strength**

(30) Priority: 03.04.2009 US 418048
(71) Applicant: Navilyst Medical, Inc., Marlborough, MA 01752-1234 (US)
(72) Inventor: Davis, Scott A., Southboro, MA 01722 (US); Lareau, Raymond, Westford, MA 01846 (US)
(74) Representative: Pfenning, Meinig & Partner GbR

(57) **Abstract**

Vascular catheters are provided that comprise a catheter shaft having one or more lumens. The catheter shaft comprises a polycarbonate polyurethane and bismuth oxychloride.

## Description

### BACKGROUND OF THE INVENTION

In current medical practice, it is commonly necessary to introduce a catheter into the vasculature for various purposes. For example, catheters may be introduced for purposes of delivering fluids, such as blood products, glucose solutions, medications, diagnostic agents, and so forth, to the vasculature. Catheters may also be introduced for purposes of withdrawing blood from the vasculature, for example, in order to treat the blood, to carry out diagnostics on the blood, and so forth. Thus, catheters must exhibit characteristics (strength, etc.) sufficient to enable them to carry out their intended functions.

### SUMMARY OF THE INVENTION

According to an aspect of the present invention, vascular catheters are provided that comprise a catheter shaft having one or more lumens. The catheter shaft comprises a polycarbonate polyurethane and bismuth oxychloride.

These and other aspects, as well as various embodiments and advantages of the present invention will become immediately apparent to those of ordinary skill in the art upon review of the Detailed Description and any claims to follow.

### BRIEF DESCRIPTION OF THE DRAWINGS

Fig 1 is a perspective view of a peripherally inserted central catheter in accordance with an embodiment of the present invention.
Fig. 2 is a bar graph depicting stress at 100% elongation for various polyurethane carbonate compositions.
Fig. 3 is a bar graph depicting stress at 100% elongation for various polyurethane carbonate compositions.
Fig. 4 is a bar graph depicting Young's Modulus for various polyurethane carbonate compositions.
Fig. 5 is a bar graph depicting Young's Modulus for various polyurethane carbonate compositions.

### DETAILED DESCRIPTION

A more complete understanding of the present invention is available by reference to the following detailed description of numerous aspects and embodiments of the invention. This detailed description of the invention is intended to illustrate but not limit the invention.

In one aspect, the present invention provides vascular catheters that comprise a catheter shaft having one or more lumens. The catheter shaft comprises a polycarbonate polyurethane and bismuth oxychloride.

The bismuth oxychloride is provided in the catheter shafts of the invention in an amount that typically ranges from approximately 5 wt% to approximately 25 wt%. The polycarbonate polyurethane is provided in an amount that typically ranges from approximately 5 wt% to approximately 75 wt%.

As used herein, a "catheter" is a medical device that includes a flexible shaft, which contains one or more lumens (including annular shafts, i.e., tubes), and which may be inserted into a subject (e.g., a vertebrate subject, for instance, a mammalian subject such a human, dog, cat, horse, etc.) for introduction of material (e.g., fluids, nutrients, medications, blood products, etc.), for removal of material (e.g., body fluids), or both.

A catheter may further include various accessory components, for example, molded components, over-molded sub-assemblies, connecting fittings such as hubs, extension tubes, and so forth. Various catheter tips designs are known, including stepped tips, tapered tips, over-molded tips and split tips (for multilumen catheters), among others.

A "venous access device" is one that provides access to the venous circulation, typically the central venous circulation (CVC) system.

A "peripheral venous catheter" is a catheter that is adapted for insertion into a peripheral vein, usually in the hand or arm.

A "central venous access catheter" is a catheter that provides access to the central venous circulation system.

Central venous access may be achieved, for instance, by direct puncture of the central venous circulation system, e.g., via the internal jugular vein, subclavian vein or femoral vein. Catheters of this type, known as "central catheters" or "central venous catheters," are relatively short, and may remain in place for months or even years.

Other central venous access catheters have also been developed which are peripheral venous catheters. These catheters can be inserted into peripheral veins (e.g., the antecubital, basilica, or cephalic vein) and advanced to access the central venous system, with the tip commonly positioned in the superior vena cava or right atrium, thus allowing for rapid dilution of infused fluids. These devices avoid difficulties associated with the direct puncture of the central venous circulation system, and are generally for short term use (e.g., a few days to a few months) to provide repeated access to a patient's vascular system, thereby avoiding multiple injections and minimizing trauma and pain to the patient.

Specific examples of catheters of this type include so-called peripherally inserted central catheters ("PICCs"), midline catheters, and peripheral catheters. A typical PICC, midline, or peripheral catheter contains a thin, flexible shaft, which contains one or more lumens and which terminates at the proximal end with a suitable fitting, such as a hub or other fitting. A primary difference between these three devices is the length of the tubing, with the peripheral catheter being the shortest and the PICC being the longest. The rationale for different lengths is driven by the type and duration of the therapy that a patient is to receive. Other differences may include a diameter, a lumen configuration, a catheter configuration, etc.

Hemodialysis catheters are another important class of central venous access catheters. Hemodialysis catheters are commonly multi-lumen catheters in which one lumen is used to carry blood from the body to a dialysis machine, and another lumen returns blood to the body. Central venous access may be attained by puncture of various major blood vessels, including the internal jugular vein, subclavian vein, or femoral vein.

Central venous access may also be provided via venous access ports. These specialized catheters typically have three components: (a) a catheter, (b) a reservoir which holds a small amount of liquid and which is connected to the catheter, and (c) a septum, which covers the reservoir and allows access to the reservoir upon insertion of a needle. The reservoir and covering septum may be surgically placed under the skin of the chest or arm, and the catheter extends into a central vein.

Catheter shafts for catheters that provide access to the central venous circulation, including those describe above, among others, are typically made from polymers. Suitable polymers are those that can be formed into a shaft, having one or more lumens, which is flexible enough to be routed through the vasculature without causing trauma to the patient. Polymeric materials that balance softness and flexibility may also be desirable. When formed into a shaft, the polymer chosen should also provide strength sufficient to ensure that the lumen does not collapse in the vasculature, and should resist repeated flexure. Recently, there has been a trend to use these devices for power injection of contrast media for use in computed tomography, requiring sufficient burst strength.

In the present invention, these properties are provided, in part, by forming catheter shafts using polyurethanes.

Polyurethanes are a family of polymers that are typically synthesized from polyfunctional isocyanates (e.g., diisocyanates, including both aliphatic and aromatic diisocyanates) and polyols, also referred to as macroglycols (e.g., macrodiols). Commonly employed macroglycols include polyester diols, polyether diols and polycarbonate diols. Typically, aliphatic or aromatic diols are also employed as chain extenders, for example, to enhance the physical properties of the material.

Polyurethanes are commonly classified based on the type of macroglycol employed, with those containing polyester glycols being referred to as polyester polyurethanes, those containing polyether glycols being referred to as polyether polyurethanes, and those containing polycarbonate glycols being referred to as polycarbonate polyurethanes. Polyurethanes are also commonly designated aromatic or aliphatic on the basis of the chemical nature of the diisocyanate component in their formulation.

In the present invention, polycarbonate polyurethanes are preferred polyurethanes, more preferably, aliphatic polycarbonate polyurethanes, although aromatic polycarbonate polyurethanes may be employed.

Macroglycols for use in forming polycarbonate polyurethanes may be selected from suitable members of the following, among others: polycarbonate diols, for example, homopolyalkylene carbonate diols and copolyalkylene carbonate diols such as those containing one or more linear or branched alkylene carbonate monomers, for instance, selected from one or more of the following: methyl carbonate, ethyl carbonate, propyl carbonate (e.g., n-propyl and isopropyl carbonate), butyl carbonate, pentyl carbonate, hexyl carbonate, heptyl carbonate, octyl carbonate, nonyl carbonate, decyl carbonate, undecyl carbonate, dodecyl carbonate, and so forth. Poly(1,6 hexyl 1,2-ethyl carbonate) diol is a common polycarbonate diol for use in forming polycarbonate polyurethanes.

Aromatic diisocyanates for use in forming polycarbonate polyurethanes may be selected from suitable members of the following, among others: 4,4'-methylenediphenyl diisocyanate (MDI), 2,4- and/or 2,6-toluene diisocyanate (TDI), 1,5-Naphthalene diisocyanate (NDI), para-phenylene diisocyanate, 3,3'-tolidene-4,4'-diisocyanate, 3,3'-dimethyl-diphenylmethane-4 and 4'-diisocyanate.

Aliphatic diisocyanates for use in forming polycarbonate polyurethanes may be selected from suitable members of the following, among others: dicyclohexylmethane-4,4'-diisocyanate (hydrongenated MDI), 1,6-hexamethylene diisocyanate (HDI), 3-isocyanatomethyl-3,5,5-trimethylcyclohexyl isocyanate (isophorone diisocyanate or IPDI), cyclohexyl diisocyanate and 2,2,4-trimethyl-1,6-hexamethylene diisocyanate.

Chain extenders for use in forming polycarbonate polyurethanes may be selected from suitable members of the following, among others: diol chain extenders such as alpha,omega-alkane diols including ethylene glycol (1,2-ethane diol), 1,3-propane diol, 1,4-butanediol, 1,5-pentane diol and 1,6-hexanediol.

Commercially available aliphatic polycarbonate polyurethanes include Carbothane® (Lubrizol Advanced Materials, Inc., Cleveland, OH, USA) and Chronoflex®AL (CardioTech International, Inc., Woburn, MA, USA). Commercially available aromatic polycarbonate polyurethanes include Bionate® (The Polymer Technology Group, Inc., Berkeley, CA, USA) and Chronoflex®AR (CardioTech International, Inc.).

Polycarbonate polyurethanes are typically thermoplastics, meaning that a variety of thermoplastic processing techniques, such as extrusion, molding, and so forth, may be employed to form medical devices and medical device components, including catheter shafts, from the same.

As noted above, when formed into a catheter shaft, the polymer chosen should also provide strength sufficient to ensure that the lumen does not collapse in the vasculature. Moreover, where used for power injection of contrast media, the selected polymer should ensure that the catheter shaft does not burst. The polymer should also resist repeated flexure.

Polycarbonate polyurethanes go a long way toward meeting these goals. They are flexible and strong, allowing catheters to be formed with thin walls, regardless of whether the catheter shaft is a single lumen shaft or a multi-lumen shaft. Subsequently, catheters made from these materials may be formed with smaller ODs as compared, for example to other catheter materials such as silicone, or they may be formed having the same OD, but with a larger ID, and therefore provide a greater flow rate.

Generally, polycarbonate polyurethanes are known to soften when exposed to elevated temperatures and aqueous environments for extended periods of time (e.g., 24 hours or more).

Bismuth compounds, such as bismuth subcarbonate, bismuth trioxide and bismuth oxychloride, among others like barium sulfate, can render a catheter shaft more absorptive of x-rays than the surrounding tissue, allowing the catheter shaft to be viewed using radiographic imaging techniques (e.g., by x-ray fluoroscopy). The amount of bismuth compound required will depend upon the thickness of the catheter wall, among other factors.

Barium sulfate and bismuth oxychloride are both used to add radiopacity to a catheter, which may be formed of a variety of thermoplastic materials. Historically, bismuth salts have not been used in polyurethane due to the potential for polymer degradation such as, for example, in the presence of bismuth subcarbonate. However, the present inventors unexpectedly found that by adding bismuth oxychloride to polycarbonate polyurethane, the softening in mechanical properties that is exhibited upon exposure to aqueous fluids at body temperature for extended time periods is markedly reduced, relative to the same polycarbonate polyurethane containing barium sulfate. This enhancement in mechanical properties (modulus and tensile strength) allows the device to resist forces exerted by routine use, including CT power injection of contrast media.
With tensile properties better retained, the need to move to unfavorably less flexible grades or thicker walls to offset the property reduction in aqueous fluids at body temperature is eliminated. Specific examples of commercially available bismuth oxychloride powders include Biron® (Merck) and PearlGlo UVR (Engelhard). Typical powder sizes may range from less than 2 to 20 microns in width.

Wall thickness for polycarbonate polyurethane central venous access catheter shafts in accordance with the invention will vary with application and may range, for example, from 0.002 inches to 0.100 inches, among other thicknesses. Other portions of the catheter may also be enhanced such as, for example, a molded suture wing, which may have a thickness of up to 0.250 inches or more.

In addition to providing enhanced mechanical properties, bismuth oxychloride also reduces surface tack and friction along the length of the catheter shaft when compared to barium sulfate. This may, for example, enhance guidewire trackability in venous access devices and enhance loading of port catheters onto port body stems. Bismuth oxychloride also produces a pearlescent surface finish which may be a desired end use desired characteristic.

Without wishing to be bound by theory, it is believed that various properties observed upon the addition of bismuth oxychloride, including strength, pearlescent finish, and lubricity characteristics, are likely due to the plate-like nature of the particles and to the thermoplastic processing techniques that are employed to form the catheter shafts, which techniques create shear forces that tend to orient the particles.

In an alternate embodiment, catheter shafts may be formed using a blend of polyurethane with polycarbonate such as Texin 4210 (Bayer Corporation, Pittsburgh, PA). As it is a polyurethane and polycarbonate blend, the addition of bismuth oxychloride will result in similarly enhanced mechanical properties.

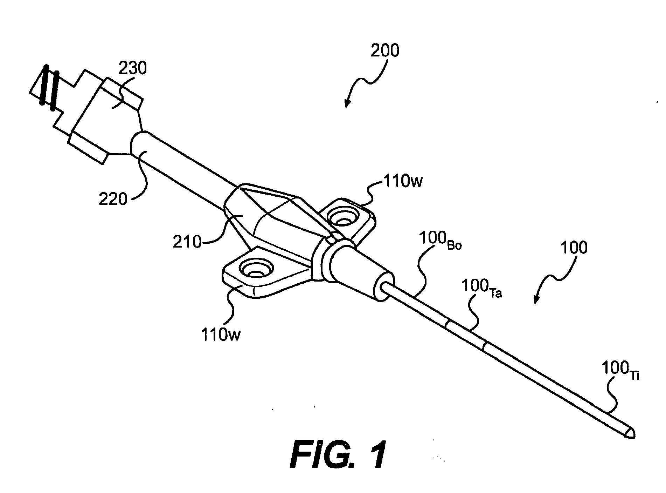

A specific example of a venous access device in accordance with the invention will now be described with reference to Fig. 1, which is a schematic perspective view of a peripherally inserted central catheter in accordance with the present invention.

The central catheter of Fig. 1 includes a catheter shaft 100 in combination with an assembly 200. Assembly 200 includes hub 210, which has suture wings 210w, extension tube 220 and a luer, which may contain a pressure activated safety valve 230 (PASV^{®}). The catheter shaft 100, which comprises extruded polycarbonate polyurethane and bismuth oxychloride in accordance with the invention, includes a body section 100_{Bo}, a tapered section 100_{Ta} and tip section 100_{Ti}. The body section 100_{Bo} has a length typically ranging from 0 to 10 cm, an outer diameter typically ranging from 0.020 to 0.262 inches, a wall thickness typically ranging from 0.002 to 0.100 inches and a durometer value ranging from 65 A to 72 D. The tip section 100_{Ti} has a length typically ranging from 0 to 80 cm an outer diameter typically ranging from 0.020 to 0.262 inches, a wall thickness typically ranging from 0.002 to 0.100 inches and a durometer value ranging from 65 A to 72 D. The tapered section 100_{Ta} has a typical length ranging from 0 to 10 cm, with outer diameter and wall thickness that transition between those of the body section 100_{Bo} and tip section 100_{Ti}. It will be understood by those of skill in the art, however, that a size of the catheter shaft 100 will vary depending on a purpose and type of catheter (e.g., PICC, port, etc.)

It will be understood by those of skill in the art that although the catheter shaft of Fig. 1 is shown with and described as having a non-tapered tip, tapered-tip catheters are also included within the scope of this invention. Additionally, although the catheter shaft shown in Fig. 1 is a single lumen shaft, shafts with multiple lumens (e.g., two, three, four, or even more) may be formed as noted above. For example a dual lumen catheter shaft may be formed and placed in an assembly along with dual extension tubes as well as an appropriate hub and a valve, if desired.

It will be apparent to those skilled in the art that various modifications and variations can be made in the structure and methodology of the present invention, without departing from the sprit and scope of the invention. Thus, it is intended that the present invention cover the modifications and variations of this invention provided that they come within the scope of the appended claims and their equivalents.

### EXAMPLES

Samples (tubular extrusions) were formed from the following: (a) 104 wt% Carbothane® PC-3585A (available from Lubrizol Advanced Materials, Inc. Cleveland, OH, USA), (b) 60 wt% Carbothane® PC-3585A and 40wt% barium sulfate, (c) 70 wt% Carbothane® PC-3585A and 30wt% bismuth oxychloride (PearlGlo UVR available from Engelhard Corp.), (c) 100 wt% Carbothane® PC-3595A, (d) 60 wt% Carbothane® PC-3595A and 40wt% barium sulfate, and (e) 70 wt% Carbothane® PC-3595A and 30wt% bismuth oxychloride. In particular, tubular extrusions of dimension 5F tip / 6F body were formed via conventional thermoplastic bump extrusion. The bismuth oxychloride was incorporated into the polyurethane prior to tube extrusion via conventional thermoplastic compounding techniques.

Stress at 100% elongation and Young's modulus were measured for the samples using an Instron Tensile Tester Model 5565. Testing was performed for as-formed samples at room temperature. Testing was also performed after removal from a conditioning bath at body temperature (37°C) for a minimum of 24 hours, immediately after removal from the water.

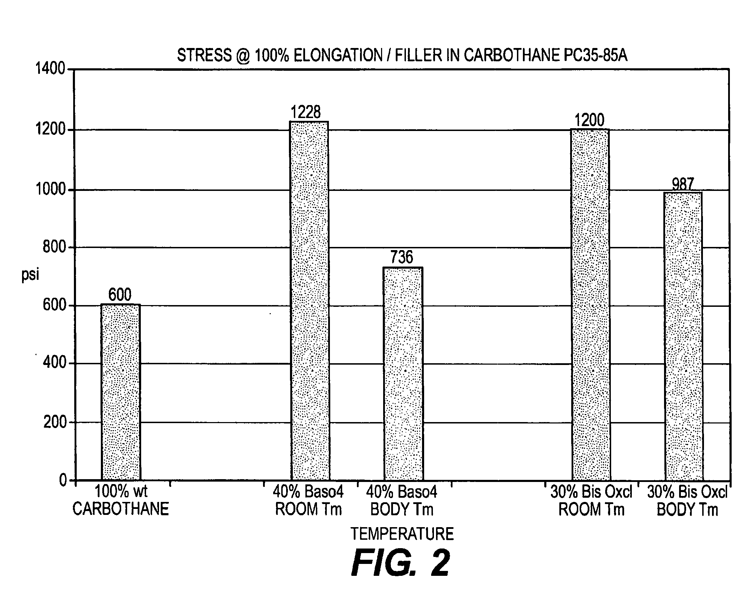

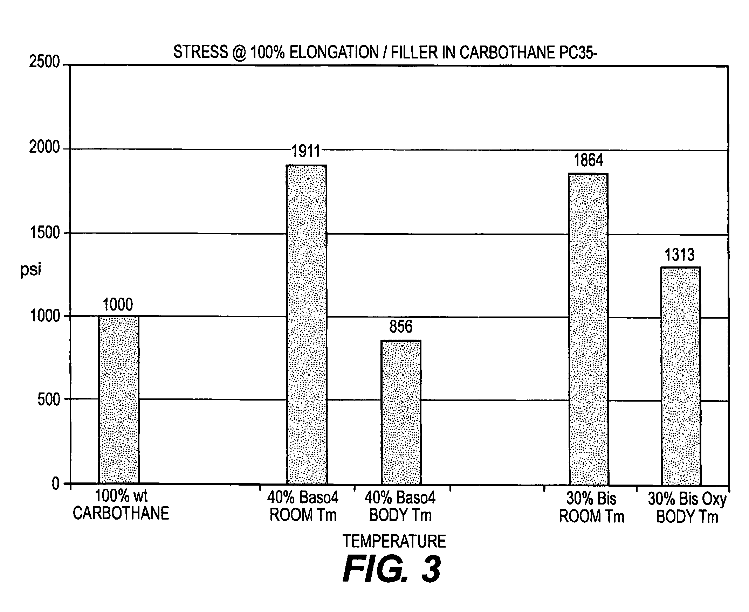

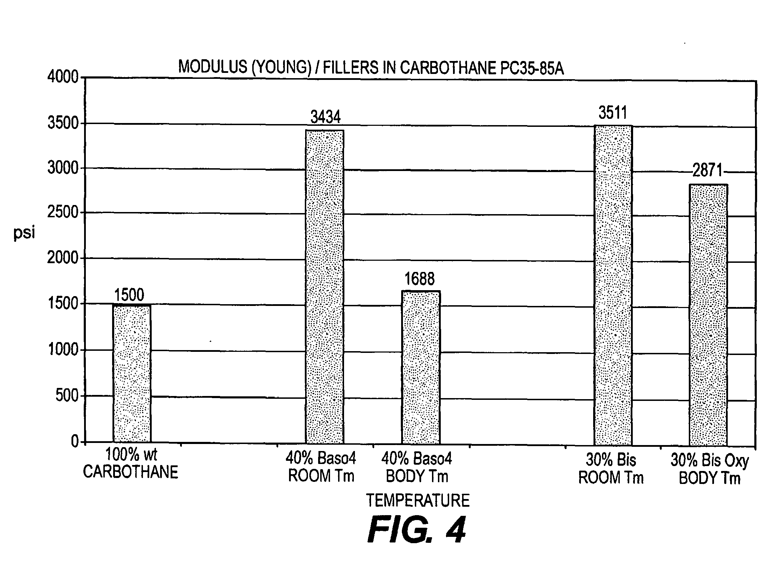

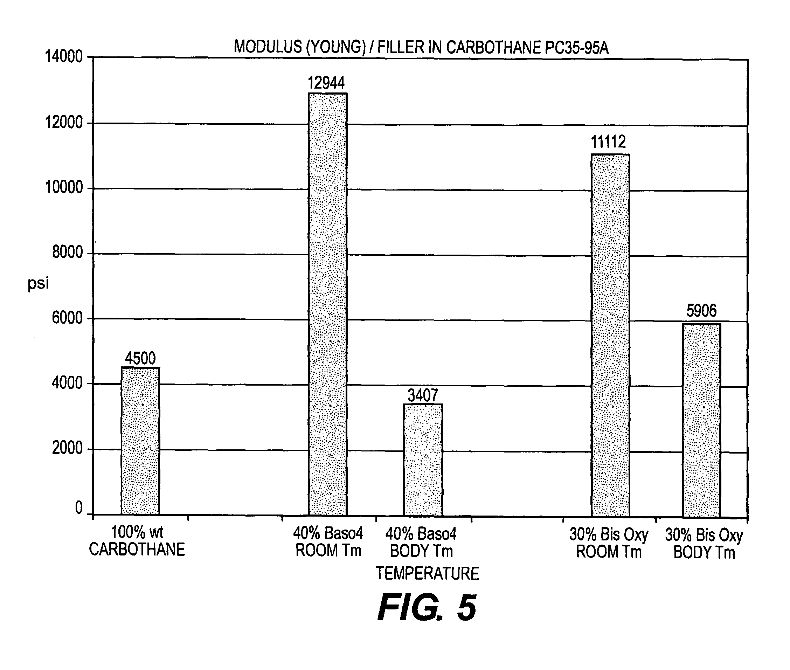

Test results are presented in Figs. 2-5. As seen from these Figures, the addition of bismuth oxychloride resulted in an unexpected increase in stress at 100% elongation and an unexpected increase Young's Modulus relative to the addition of traditional barium sulfate. This was observed for both grades of Carbothane® and was observed both at room temperature and post-conditioning in water at body temperature. It should be noted that the room temperature stress at 100% elongation and Young's Modulus did not increase with the addition of bismuth oxychloride as compared to traditional barium sulfate.

In particular, with bismuth oxychloride in Carbothane® PC-3585A there is only a 17.8% reduction in stress at 100% elongation upon conditioning versus a 40% reduction with barium sulfate. With bismuth oxychloride in Carbothane® PC-3585A there is only an 18.2% reduction in Young's Modulus upon conditioning versus a 50.8% reduction with barium sulfate. With bismuth oxychloride in Carbothane® PC-3535A there is only a 29.5% reduction in stress at 100% elongation upon conditioning versus a 55% reduction with barium sulfate. With bismuth oxychloride in Carbothane® PC-3595A there is only a 46.8% reduction in Young's Modulus upon conditioning versus a 73.7% reduction with barium sulfate.

Although various embodiments are specifically illustrated and described herein, it will be appreciated that modifications and variations of the present invention are covered by the above teachings and are within the purview of any appended claims without departing from the spirit and intended scope of the invention.

## Claims

1. A central venous access device comprising a catheter shaft that comprises polycarbonate polyurethane and bismuth oxychloride.

2. The central venous access device of claim 1, wherein the catheter shaft comprises 60 to 90 wt% polycarbonate polyurethane and 10 to 40 wt% bismuth oxychloride.

3. The central venous access device of claim 1, wherein the polycarbonate polyurethane is an aliphatic polycarbonate polyurethane.

4. The central venous access device of claim 1, wherein the catheter shaft comprises a single lumen.

5. The central venous access device of claim 1, wherein the catheter shaft comprises multiple lumens.

6. The central venous access device of claim 1, wherein the device is a central catheter.

7. The central venous access device of claim 1, wherein the device is a peripheral catheter.

8. The central venous access device of claim 1, wherein the device is a midline catheter.

9. The central venous access device of claim 1, wherein the device is a hemodialysis catheter.

10. The central venous access device of claim 1, wherein the device is a peripherally inserted central catheter.

11. The central venous access device of claim 10, wherein the catheter shaft has a diameter between 2 and 20F.

12. The central venous access device of claim 1, wherein the device is a venous access port.

13. The central venous access device of claim 1, wherein the catheter shaft is an extruded catheter shaft.

14. The central venous access device of claim 1, wherein the catheter shaft has a durometer value between 65A and 72D.
